# DEMANDE DE BREVET EUROPEEN

(11) **EP 3 617 968 A1**
(43) Date de publication de la demande: **04.03.2020**
(21) Numéro de dépôt: 19194459.4
(22) Date de dépôt: 29.08.2019
(51) Int. Cl.: G06Q 10/06, G08B 13/14

(54) **PROCÉDÉ ET DISPOSITIF DE SURVEILLANCE D'UN LIQUIDE CONTENU DANS UN CONTENANT**

(30) Priorité: 30.08.2018 FR 1857810
(71) Demandeur: CONNECTED SHIFTS, 92320 Chatillon (FR)
(72) Inventeur: CHERPANTIER, Frédéric, 92500 RUEIL-MALMAISON (FR); MOULIN, Nicolas, 92320 CHATILLON (FR)
(74) Mandataire: Cornuejols, Georges

(57) **Abrégé**

Le dispositif (12) de surveillance d'un liquide contenu dans un contenant (11) comporte :
- un capteur (13) d'au moins une valeur de grandeur physique d'environnement du contenu, fournissant, à une fréquence d'échantillonnage, des valeurs captées,
- un moyen (14) de communication, à une fréquence de transmission, d'information représentative des valeurs captées de chaque grandeur physique,
- un capteur (15) d'état du contenant configuré pour détecter un changement d'état du contenant, et
- un contrôleur (16) configuré :
- pour réaliser un apprentissage de plages de valeurs de grandeurs physiques captées pendant une période prédéterminée suivant un détection de changement d'état, période ne comportant pas de détection de changement d'état, le moyen de communication étant configuré pour transmettre un signal d'alarme, après cette période d'apprentissage, lorsqu'au moins une valeur de grandeur physique captée se trouve en dehors de la plage de grandeurs physiques apprise ;
et / où
- pour détecter la sortie du liquide du contenant, le moyen de communication étant configuré pour transmettre une information représentative de la détection de la sortie du liquide.

## Description

### DOMAINE DE L'INVENTION

La présente invention concerne un procédé et un dispositif de surveillance d'un liquide contenu dans un contenant. Elle s'applique à la supervision et la sécurisation des parcours (transport, stockage, position et mouvement dans l'espace, consommation) et des conditions de vie (température, hygrométrie, lumière, pression) de bouteilles de vin, spiritueux et, plus généralement, de tous fluides transportés en bouteilles, flacons ou poches fermées : liquides consommables (eau, sodas, etc.), parfums, poches de sang, bonbonnes de gaz, etc.

### ETAT DE LA TECHNIQUE

On connaît des dispositifs et systèmes de surveillance de la chaîne du froid dans l'agro-alimentaire. Le dispositif « Cellier Domesticus » (marque déposée) ressemble à une bouteille en bois à insérer dans un cellier et comporte un thermomètre et un hygromètre connectés permettant de suivre, à distance, la température et le taux d'humidité du cellier. Un climatiseur optionnel assure le maintien de la température du cellier dans une plage de valeurs définie par l'utilisateur.

Avec le dispositif « eProvenance » (marque déposée), les données sont collectées à la caisse de vin et pas à la bouteille. L'autonomie du capteur est très faible (de l'ordre de deux ans maximum). De plus, ce dispositif ne communique pas à plusieurs mètres, ce qui interdit un suivi en temps réel, des alertes, ...

Ces différents capteurs, pour fonctionner de manière autonome sur une longue durée, possèdent une alimentation électrique externe (secteur, panneau solaire, effet Peltier, ensemble de batteries, etc.). S'ils ne possèdent pas d'alimentation par apport externe d'énergie, soit ils ne durent pas suffisamment longtemps (moins de trois ans), par rapport à la durée de vie du contenu des bouteilles (10 ans, 20 ans, ou plus), soit ils durent suffisamment longtemps mais sans export régulier par transmission à distance des données captées au cours de leurs mesures.

Ces dispositifs ne permettent donc pas de fourniture automatique et sécurisée de données régulières collectées à distance sur un ensemble significatif de bouteilles ou de poches à surveiller, par exemple plusieurs centaines, milliers ou millions, pendant toute leur durée de vie.

### OBJET DE L'INVENTION

La présente invention vise à remédier à tout ou partie de ces inconvénients.

A cet effet, selon un premier aspect, la présente invention vise un dispositif de surveillance d'un liquide contenu dans un contenant, qui comporte :
- un capteur d'au moins une valeur de grandeur physique d'environnement du contenu, fournissant, à une fréquence d'échantillonnage, des valeurs captées,
- un moyen de communication, à une fréquence de transmission, d'information représentative des valeurs captées de chaque grandeur physique,
- un capteur d'état du contenant configuré pour détecter un changement d'état du contenant, et
- un contrôleur configuré :
   - pour réaliser un apprentissage de plages de valeurs de grandeurs physiques captées pendant une période prédéterminée suivant un détection de changement d'état, période ne comportant pas de détection de changement d'état, le moyen de communication étant configuré pour transmettre un signal d'alarme, après cette période d'apprentissage, lorsqu'au moins une valeur de grandeur physique captée se trouve en dehors de la plage de grandeurs physiques apprise ;
      et / où
   - pour détecter la sortie du liquide du contenant, le moyen de communication étant configuré pour transmettre une information représentative de la détection de la sortie du liquide.

Grâce à ces dispositions, l'apprentissage permet au dispositif de déterminer automatiquement les conditions habituelles de conservation du contenant et du contenu et donc de détecter plus finement les événements sortant de cette conservation habituelle et/ou la détection de sortie du liquide peut être connue à distance.

Dans des modes de réalisation, le contrôleur est configuré pour changer de fréquence d'échantillonnage et/ou de fréquence de transmission après détection d'un changement d'état par le capteur d'état.

Grâce à ces dispositions, lorsque le capteur d'état détecte que l'état du contenant est modifié, par exemple lorsque le contenant est déplacé ou séparé du dispositif, le contrôleur modifie la fréquence d'échantillonnage et/ou de transmission d'information, ce qui permet de suivre fidèlement l'environnement dans lequel se trouve le contenant. En effet, lors d'un déplacement ou d'une séparation du contenant et du dispositif, le contenant risque d'être soumis à des variations d'environnement plus rapides, ce qui implique que le contenu se trouvant dans ce contenant risque d'être détérioré.

Dans des modes de réalisation, le dispositif comporte une horloge pour fournir au moyen de communication la fréquence de transmission et une alimentation électrique configurée pour n'alimenter de manière continue que le contrôleur, le capteur d'état et l'horloge, chaque capteur de valeurs de grandeur physique et le moyen de communication n'étant alimentés que par intermittence.

Grâce à ces dispositions, la consommation d'énergie est très réduite en dehors des instants de capture et/ou de transmission de valeurs de grandeurs physiques.

Dans des modes de réalisation, l'alimentation électrique est une pile au lithium - chlorure de thionyle et/ou qui présente les caractéristiques suivantes :
- elle présente une consommation permanente inférieure à 10 nA ;
- elle supporte des pics de courant de 50mA et
- elle ne présente pas d'autodécharge.

Grâce à ces dispositions, l'autodécharge naturelle ou les courants de fuite de la pile sont très réduits et la durée de vie de la pile est prolongée en conséquence.

Dans des modes de réalisation, le capteur d'état est configuré pour détecter un déplacement du contenant. Par exemple, le capteur d'état comporte un accéléromètre.

Grâce à ces dispositions, un déplacement du contenant est détecté et la fréquence d'échantillonnage et/ou de transmission est modifiée.

Dans des modes de réalisation, le capteur d'état est configuré pour détecter la séparation du dispositif, d'une part, du contenant, d'autre part.

Grâce à ces dispositions, la séparation du dispositif et du contenant est détectée et une alerte peut rapidement être déclenchée.

Dans des modes de réalisation, le contrôleur est configuré pour augmenter la fréquence d'échantillonnage et la fréquence de transmission après détection d'un déplacement du contenant.

Grâce à ces dispositions, la connaissance, à distance, de l'environnement du contenant et/ou du contenu est plus rapidement connue et plus souvent mise à jour.

Dans des modes de réalisation, le contrôleur est configuré :
- pour réaliser un apprentissage de plages de valeurs de grandeurs physiques captées pendant une période prédéterminée suivant une détection de changement d'état, période ne comportant pas de détection de changement d'état et
- pour transmettre un signal d'alarme, après cette période d'apprentissage, lorsqu'au moins une valeur de grandeur physique captée se trouve en dehors de la plage de grandeurs physiques apprise.

Grâce à ces dispositions, le dispositif peut apprendre les variations normales de l'environnement du contenant, pendant une période d'apprentissage, puis déclencher une alerte au cas où une variation anormale de l'environnement est détectée.

Dans des modes de réalisation, le capteur d'au moins une valeur de grandeur physique d'environnement du contenant comporte des capteurs de valeurs de grandeurs physiques représentative de la température, de l'hygrométrie, de l'éclairement et de la pression atmosphérique de l'environnement du contenant.

Grâce à ces dispositions, les principaux facteurs de détérioration du contenu sont surveillés.

Dans des modes de réalisation, le contrôleur est configuré pour détecter la sortie du liquide du contenant, le moyen de communication étant configuré pour transmettre une information représentative de la détection de la sortie du liquide.

Grâce à ces dispositions, le gestionnaire des contenants et/ou le producteur des contenant peuvent connaître et étudier la durée de vie des liquides et optimiser leur gestion ou production en fonction de cette information.

Selon un deuxième aspect, la présente invention vise un procédé de surveillance d'un liquide contenu dans un contenant, qui comporte :
- une étape de capture d'au moins une valeur de grandeur physique d'environnement du contenu, fournissant, à une fréquence d'échantillonnage, des valeurs captées,
- une étape de communication, à une fréquence de transmission, d'information représentative des valeurs captées de chaque grandeur physique,
- une étape de capture d'état du contenant configuré pour détecter un changement d'état du contenant ; et
   - une étape d'apprentissage de plages de valeurs de grandeurs physiques captées pendant une période prédéterminée suivant un détection de changement d'état, période ne comportant pas de détection de changement d'état et une étape de transmission d'un signal d'alarme, après cette période d'apprentissage, lorsqu'au moins une valeur de grandeur physique captée se trouve en dehors de la plage de grandeurs physiques apprise ;
      et/ou
   - une étape de détection de la sortie du liquide du contenant et une étape de communication d'une information représentative de la détection de la sortie du liquide.

Les avantages, buts et caractéristiques particulières de ce procédé étant similaires à ceux du dispositif objet de l'invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du dispositif de surveillance et de l'assemblage objet de la présente invention, en regard des dessins annexés, dans lesquels :
- la figure 1 représente, schématiquement, une bouteille munie d'un mode de réalisation particulier du dispositif objet de l'invention,
- la figure 2 représente, schématiquement, un cycle de vie de modes de réalisation particuliers du dispositif objet de l'invention,
- la figure 3 représente, schématiquement, le fonctionnement au cours de période de stockage du dispositif illustré en figure 1,
- la figure 4 représente, schématiquement, le fonctionnement au cours de période de transport du dispositif illustré en figure 1,
- la figure 5 représente, schématiquement, une machine à états globale représentative du fonctionnement du dispositif illustré en figure 1 et
- la figure 6 représente, sous forme d'un logigramme, des étapes d'un mode de réalisation particulier du procédé objet de l'invention.

### DESCRIPTION DETAILLEE DE MODES DE REALISATION DE L'INVENTION

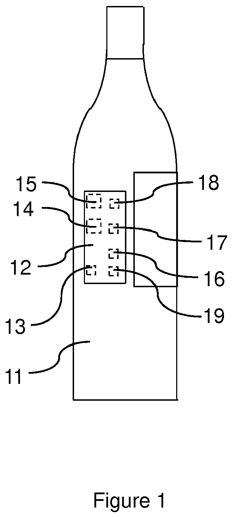

On observe, en figure 1, un dispositif 12 de surveillance d'un liquide contenu dans un contenant 11, prenant ici la forme d'une bouteille. Ce dispositif 12 comporte :
- un capteur 13 d'au moins une valeur de grandeur physique d'environnement du contenu, notamment du contenant 11, fournissant, à une fréquence d'échantillonnage, des valeurs captées,
- un moyen 14 de communication, à une fréquence de transmission, d'information représentative des valeurs captées de chaque grandeur physique d'environnement,
- un capteur 15 d'état du contenant 11 configuré pour détecter un changement d'état du contenant et
- un contrôleur 16.

Préférentiellement, le contrôleur 16 commande la fréquence d'échantillonnage du capteur 13 et/ou la fréquence de transmission du moyen 14 de communication.

Par ailleurs, le dispositif 12 comporte :
- une alimentation électrique 17,
- une horloge 18 et
- une mémoire 19.

Le contenant 11 peut prendre différentes formes, par exemple bouteilles, flasques, fioles, flacons ou poches. Ce contenant 11 contient, généralement, un liquide dont les propriétés peuvent évoluer différemment en fonction de l'environnement du contenu, notamment du contenant 11 (chaleur, choc thermique, humidité, éclairement, pression par exemple). Ce liquide est, par exemple, un vin ou un spiritueux, un parfum, un liquide physiologique, du sang, un principe actif, un extrait, un médicament, ...

Le capteur 13 peut mesurer, par exemple, la température, l'hygrométrie, l'éclairement et/ou la pression atmosphérique de l'environnement du contenant 11. Le capteur 13 fournit un signal représentatif de chaque valeur de grandeur physique captée.

Le moyen de communication 14 utilise préférentiellement des ondes radio, par exemple les ondes utilisées pour la transmission numérique bas débit pour optimiser la consommation électrique ou encore les ondes utilisées pour une communication locale (par exemple selon l'un des protocoles WiFi ou Bluetooth, marques déposées). Le moyen de communication 14 fournit ainsi à un serveur central (non représenté) des messages représentatifs des valeurs de grandeur physique captées.

Préférentiellement, le moyen de communication 14 code les informations à transmettre en les comprimant pour réduire la quantité de données à transmettre. Ce codage s'effectue, par exemple, par une transformation mathématique non-linéaire qui a pour buts :
- de conserver une variation dynamique importante, donc une grande précision, dans la zone de variation attendue des valeurs des paramètres d'environnement ;
- d'indiquer les excursions des valeurs de ces variables d'environnement en dehors de la zone attendue, avec une précision moindre ;
- de minimiser le volume des informations à transmettre car le système de transmission utilisé impose des messages courts (en nombre d'octets) et peu fréquents (1 message toutes les 10 minutes par exemple) ; et
- de transmettre plusieurs relevés des valeurs de paramètres d'environnement en un seul message.

Dans la pratique et à titre d'exemple, trois méthodes distinctes de compression des données sont décrites permettant de transmettre, dans l'exemple, 10 valeurs de température relevées à dix instants différents :
- Méthode 1 : transmettre 10 valeurs codées sur 10 bits chacune (donc 100 bits au total), permettant une dynamique de -20,0°C à +60,0°C, au dixième de degré près ;
- Méthode 2 : transmettre 10 bits représentant la valeur moyenne des 10 valeurs ainsi que 10 nombres relatifs avec une dynamique faible (+/- 3,0°C) codés sur 6 bits représentant l'écart de chaque mesure à la moyenne. Un tel message comporte 70 bits soit 30% de moins que la méthode 1 ;
- Méthode 3 : transmettre 10 bits représentant la valeur moyenne des 10 valeurs ainsi que 2 nombres relatifs codés sur 6 bits représentant l'écart à la moyenne de la plus grande et de la plus faible des valeurs mesurées. Dans ce dernier cas, la taille du message tombe à 22 bits soit 68% de moins que la méthode 2.

Ces méthodes de compression des données transmises sont particulièrement efficaces dans l'utilisation du dispositif puisque :
- on s'intéresse à une dynamique faible (quelques degrés de température autour d'une valeur moyenne) ;
- les seules valeurs intéressantes sont les valeurs extrêmes (la plus grande ou la plus petite), dans la plage dynamique réduite ;
- il n'est pas important de quantifier finement l'ampleur de la déviation lorsqu'une valeur sort de la dynamique étudiée.

Le capteur d'état 15 est préférentiellement configuré pour détecter un déplacement du contenant 11. Par exemple, le capteur d'état 15 est un accéléromètre, par exemple sous forme d'un dispositif micro électromécanique (Micro electromechanical device, en anglais). Un signal de détection de mouvement ou déplacement est émis par le capteur d'état 15 à destination du contrôleur 16 lorsqu'une variation de l'accélération sur l'un des trois axes est supérieure à une valeur limite.

Dans des modes de réalisation, le capteur d'état 15 est configuré pour détecter la séparation du dispositif 12, d'une part, du contenant 11, d'autre part, éventuellement en plus de la détection de déplacement. Par exemple, le capteur d'état est alors un contacteur prenant la forme d'un circuit électrique fragile collé sur le contenant 11 et qui se déchire en cas de traction sur le dispositif 12 en vue de son arrachement.

Le contrôleur 16, par exemple un processeur, est préférentiellement configuré pour changer, préférentiellement augmenter, la fréquence d'échantillonnage et la fréquence de transmission après détection d'un changement d'état du contenant, par exemple déplacement du contenant ou séparation du dispositif 12.

Préférentiellement, le contrôleur 16 est configuré :
- pour réaliser un apprentissage de plages de valeurs de grandeurs physiques captées pendant une période prédéterminée suivant un détection de changement d'état, période ne comportant pas de détection de changement d'état et
- pour transmettre un signal d'alarme, après cette période d'apprentissage, lorsqu'au moins une valeur de grandeur physique captée se trouve en dehors de la plage de grandeurs physiques apprise.

L'apprentissage est réalisé en traitant statistiquement les valeurs captées pendant cette phase. Par exemple, pour chaque grandeur physique, la plage de valeurs ne déclenchant pas l'émission d'un signal d'alarme s'étend de la valeur moyenne moins n fois l'écart-type jusqu'à la valeur moyenne plus m fois l'écart-type, n et m étant des nombres, par exemple 2 ou 3.

Optionnellement, des analyses de cofacteurs sont réalisées, une combinaison de valeurs captées de grandeurs physiques permettant l'établissement d'une plage de valeurs ne déclenchant pas l'émission d'un signal d'alarme. Par exemple, la température, l'hygrométrie, l'éclairement et/ou la pression atmosphérique peuvent être liés et varier ensemble selon les saisons ou la météorologie. Une combinaison linéaire des valeurs captées peut compenser cette variation pour fournir une valeur sensiblement constante pendant l'année. On peut alors établir la plage de valeurs pour cette combinaison, complémentairement ou en substitution des plages de valeurs associées aux différentes grandeurs physiques captées.

L'horloge 18 est, par exemple, de type RTC (acronyme de Real Time Clock, pour horloge temps réel). Elle fournit au contrôleur 16 la fréquence de réveil. L'alimentation électrique 17 est configurée pour n'alimenter de manière continue que le contrôleur 16, le capteur d'état 15 et l'horloge 18. Ainsi, chaque capteur 13 de valeurs de grandeur physique et le moyen de communication 14 ne sont alimentés que par intermittence, lorsque le contrôleur 16 les active.

L'alimentation électrique 17 possède les caractéristiques suivantes, - elle peut être, par exemple, une pile au lithium - chlorure de thionyle :
- elle permet une consommation permanente très faible (de l'ordre de quelques nA), préférentiellement inférieure à 10 nA ;
- elle supporte des pics de courant en consommation lorsque le système de transmission est mis en service, préférentiellement jusqu'à des pics de 50mA. Il n'est pas rare que des pics entre 30 et 50mA de consommation soient générés par la mise en route d'un système de transmission radio (exemple Sigfox marque déposée, ou LoRa marque déposée). La pile est choisie de telle sorte que ces pics n'altèrent pas sa chimie et ne diminuent pas significativement sa capacité ;
- elle ne souffre pas d'autodécharge. La plupart des piles et accumulateurs s'auto-déchargent même en l'absence de consommation. Ce mécanisme d'autodécharge peut atteindre 2 à 3% par mois selon les éléments chimiques constitutifs de la pile. Cet ordre de grandeur n'est pas acceptable dans une application devant fonctionner pendant plusieurs années ;
- elle a une densité volumique d'énergie permettant des dimensions compatibles avec les faibles encombrements requis.

Ainsi, l'architecture électronique est étudiée pour pouvoir ne laisser sous tension permanente que les éléments vitaux du système, c'est-à-dire ceux qui permettent d'assurer le réveil du système (processeur 16, horloge 18, accéléromètre et, éventuellement contacteur 15). Les autres éléments (capteurs 13 hors accéléromètre ou contacteur, mémoire 19, système de transmission 14) ne sont donc pas alimentés en permanence.

Il n'existe que deux facteurs permettant de réveiller le système lorsqu'il est en sommeil :
- le réveil à cause d'une mise en mouvement ou d'arrachement (qui se traduit en électronique par un signal d'interruption venant de l'accéléromètre ou du contacteur 15 et adressé au processeur 16) ou, éventuellement, d'un arrachement ;
- le réveil programmé au bout d'un certain temps, paramétrable, de sommeil (qui se traduit en électronique par une interruption venant de l'horloge 18).

Ainsi, seuls le processeur 16, l'accéléromètre et/ou contacteur 15 et l'horloge 18 sont alimentés en permanence. Le processeur 16 pilote l'alimentation des autres composants en fonction de ses besoins. La conséquence directe est que la consommation au repos des autres éléments (capteurs 13 hors accéléromètre et contacteur, système de transmission 14, mémoire 19) n'intervient plus dans la détermination de la longévité de l'alimentation électrique 17.

Le dispositif 12 peut se trouver dans quatre configurations d'alimentation dont les consommations sont différentes :
- Configuration1 : le dispositif 12 est éteint, donc non alimenté. Dans ce cas, les seuls paramètres qui influent sur l'autonomie du dispositif sont les courants de fuite et l'autodécharge de la source d'alimentation 17 ;
- Configuration 2 : le dispositif 12 est en veille : seuls le processeur 16, l'horloge 18 et le capteur d'état 15 sont alimentés. Dans cette configuration, le dispositif 12 est passif, sa consommation est réduite, et il ne peut réagir qu'aux événements du capteur d'état 15 ou de l'horloge 18 (réveil périodique du dispositif 12 après une période d'endormissement programmé) ;
- Configuration 3 : le dispositif 12 active chaque capteur d'environnement 13 afin d'effectuer une mesure de paramètre d'environnement. Dans ce cas, le processeur 16 est activé, chaque capteur d'environnement 13 et une mémoire 19 qui sert à stocker les valeurs captées des grandeurs mesurées sont alimentés. Cette troisième configuration n'est utilisée que pendant la durée de l'acquisition et de mémorisation des grandeurs mesurées par chaque capteur d'environnement 13. Sitôt la mesure effectuée, le dispositif 12 revient dans un état de moindre consommation, la configuration 2 ;
- Configuration 4 : lorsque le dispositif 12 transmet des valeurs à un serveur central, par l'intermédiaire du moyen de communication 14, le moyen de communication 14 est alimenté durant toute la période de transmission, et jusqu'à la transmission complète du message constitué par le processeur à destination du serveur central. Cette configuration est en général la plus gourmande en énergie. Sitôt la transmission effectuée, le dispositif revient dans un état de moindre consommation.

Les deux paramètres principaux du fonctionnement du dispositif 12 qui influent sur la consommation d'énergie sont donc :
- la fréquence d'échantillonnage qui est la fréquence à laquelle chaque capteur d'environnement 13 est alimenté pour acquérir une valeur instantanée de la grandeur que le capteur 13 mesure. Le dispositif 12 acquiert et mémorise ainsi une valeur mesurée par le capteur à chaque période d'échantillonnage ; et
- la fréquence de transmission : c'est la fréquence à laquelle les valeurs mesurées et mémorisées sont transmises à un serveur central.

Dans toute la description, on utilise les définitions suivantes :
- Période : durée déterminée.
- Etat : caractéristique relative et temporelle (c'est-à-dire associée à une période), comprenant des plages de paramètres physiques. Cette caractéristique a de plus la propriété suivante : les paramètres concernés ont une stabilité particulière au sein de la période considérée. Ainsi un « état » correspond à un état matériel du dispositif de surveillance (y compris état des mémoires mais pas leur contenu qui peut évoluer dans le même état matériel) ;
- Etape : Intervalle de temps pendant lequel le dispositif ne change pas d'état et pendant lequel des opérations sont effectuées par les éléments matériels du dispositif de surveillance (notamment étapes de capture, calcul et transmission de données) ;
- Phase : intervalle de temps de la vie d'un contenant ou d'un contenu ; une phase comporte plusieurs étapes (une étape est uniquement dans une et une seule phase). Ainsi une « phase » est liée au contenant ou au contenu (ex : phase de vie, y compris transport) ;
- Mode : Succession d'états permettant l'adaptation du comportement d'un dispositif en réaction à la phase dans laquelle se trouve le contenant qu'il équipe.
- Configuration : état de l'alimentation électrique du dispositif de surveillance.

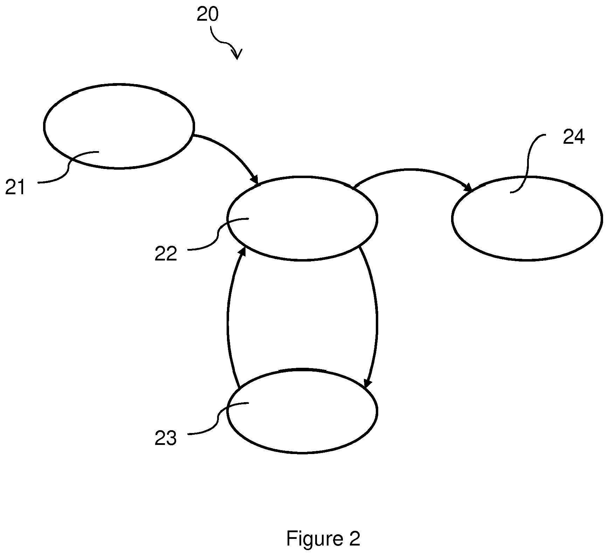

On observe, en figure 2, quatre phases 21 à 24 du cycle de vie du dispositif 12.

Dans la phase initiale 21, le dispositif 12 est dans l'état initial par défaut lorsque le dispositif 12 est fabriqué. Dans cet état initial, le dispositif 12 n'est pas alimenté, n'est pas utilisé et n'est pas associé à un contenant 11. Cette phase initiale 21 se prolonge tant que le dispositif 12 est stocké. Dans cette phase initiale 21, le dispositif 12 ne mesure aucune valeur de paramètre d'environnement ou d'état et ne transmet aucune information. Sa consommation électrique est donc nulle, à l'exception de l'autodécharge naturelle de la pile ou des courants de fuite.

La phase d'activation commence à partir du moment où on associe le dispositif 12 à un contenant 11. Le dispositif 12 est installé sur le contenant 11 par un opérateur, ce qui active le contacteur de séparation du dispositif et déclenche son alimentation électrique. Dans cette phase d'activation, le dispositif 12 indique son état opérationnel (par exemple par une source lumineuse qui s'active et qui permet à l'opérateur de constater le bon fonctionnement du dispositif 12). Le dispositif 12 commence à surveiller les valeurs de paramètres d'environnement du contenu, dont l'état du contenant 11, par l'intermédiaire des capteurs d'environnement 13 et d'état 15, respectivement.

La consommation du dispositif 12 est significative puisqu'il vérifie la présence et la disponibilité de l'ensemble de ses capteurs 13 et 15 et périphériques. En particulier, le dispositif 12 vérifie sa capacité à transmettre des informations à un serveur central en utilisant le moyen de communication 14. Cette première transmission permet au système central d'associer un identifiant du dispositif 12 (identifié par un numéro de série unique) au contenant 11 auquel le dispositif 12 est lié (château, cuvée, millésime, ...). La durée de cette phase d'activation est courte puisqu'une fois la première transmission effectuée, le dispositif 12 entre en mode de transport. La vie du contenant 11 entre l'activation du dispositif 12 et la consommation du contenu est une alternance entre des phases de transport 22 et des phases de stockage 23.

La phase de stockage 23 se décompose en trois modes :
Le mode d'apprentissage est celui dans lequel le dispositif 12 mesure, d'abord, les valeurs habituelles des paramètres d'environnement de l'espace de stockage du contenant. Le contrôleur réalise alors l'étape d'apprentissage décrite ci-dessus.

Le mode endormissement est celui qui suit immédiatement le mode apprentissage lorsque celui-ci prend fin.

Le dispositif 12 passe dans le mode d'ajustement pour l'une ou l'autre des deux raisons suivantes :
- la durée prévue du mode d'endormissement est écoulée ;
- au moins une des valeurs des paramètres d'environnement est sortie de la plage de variation calculée en fonction des valeurs habituelles des paramètres d'environnement.

Dans le mode d'ajustement, on procède à un ajustement du calcul de la plage de variation des valeurs des paramètres d'environnement. L'état ajustement est donc, de ce point de vue, similaire à l'état apprentissage.

Dans la phase de transport 22, l'état d'alarme est l'état du dispositif 12 lorsqu'au moins un des paramètres d'environnement surveillés présente une valeur captée qui se trouve en dehors de la plage de variation calculée. Dans ce cas, préférentiellement, la fréquence d'échantillonnage est augmentée et la fréquence de transmission est augmentée à sa valeur maximale. Cet état d'alarme dure tant qu'au moins une valeur de paramètre d'environnement est en dehors de la plage de variation calculée.

La phase de consommation 24 correspond à la fin de vie du contenu du contenant 11. Chaque capteur d'environnement ainsi que le capteur d'état permettent de détecter certains événements de la vie du contenu et de son contenant.

Cette phase de consommation correspond à la sortie du contenu du contenant. Par apprentissage automatique ou analyse statistique, le concepteur du dispositif détermine des valeurs d'environnement qui accompagnent un déplacement du contenant lors de cette phase terminale. La position du contenant par rapport à l'horizontale est un des facteurs d'identification du déroulement de cette phase terminale. Un autre facteur d'identification de cette phase terminale est la réduction de la quantité de contenu dans le contenant, estimée, par exemple, par la diminution de l'absorbance du contenu à l'intérieur du contenant (rapport de l'éclairement mesuré à l'intérieur du contenant sur l'éclairement mesuré à l'extérieur du contenant). Les valeurs limites pour ces paramètres (horizontalité et absorbance) sont déterminées comme pour l'étape d'apprentissage exposé ci-dessus, mais sur des bouteilles tests, en laboratoire, et non au cours de l'utilisation du dispositif sur une bouteille.

La détection de sortie (ou détection de consommation) du contenu s'effectue par un dispositif de surveillance de fluide équipé de capteurs d'environnement tels que décrits ci-dessus, appliqué au contenant.

Par exemple, pour la consommation du vin contenu dans une bouteille : augmentation de température, augmentation de luminosité externe et interne au contenant, position verticale entrecoupée de déplacements en position horizontale, rotation de la bouteille sur son axe lors de la découpe de la capsule, signature vibratoire de l'extraction du bouchon, signature vibratoire de l'écoulement du vin.

Au cours de la recherche de détection de sortie du contenu, on applique une fréquence d'échantillonnage propre à permettre cette détection : cette fréquence peut augmenter progressivement depuis celle définie pour la phase de transport 22 jusqu'à atteindre une captation par centième de seconde.

Le principe de cette détection est de calculer une probabilité concluante de consommation du contenu par un faisceau d'indices obtenus par des mesure effectuées par différents capteurs accompagnant habituellement la consommation du contenu provenant du contenant, alors que chaque indice pris individuellement peut ne pas garantir une détection fiable.

On note qu'il n'est généralement pas nécessaire de détecter l'instant précis où se fait la consommation du contenu mais on vise à détecter une préparation de consommation et des phénomènes qui accompagnent généralement cette consommation, soit qu'ils précèdent soit qu'ils suivent cette consommation de quelques minutes ou de quelques heures.

Certains des critères ou indices de détection décrits ci-dessous bénéficient d'une forte augmentation de la fréquence d'échantillonnage, pouvant atteindre une captation par centième de seconde. Préférentiellement, on augmente donc la fréquence d'échantillonnage au fur et à mesure que certains des indices augmentent la probabilité de détecter la consommation du contenu de la bouteille. Le niveau de réalisation de l'ordre chronologique de survenance des indices est pris en compte dans la détermination de la détection de sortie du liquide.

Pour les vins rouges, les indices de détection comportent au moins une, préférentiellement au moins deux, des détections suivantes :
I. le dispositif est dans une phase de transport : la consommation de la bouteille s'accompagne systématiquement de mouvements de la bouteille ;
II. la température mesurée par le dispositif augmente d'au moins une valeur prédéterminée, par exemple un degré Celsius) : la consommation de la bouteille se fait plutôt dans un environnement tempéré (salle à manger, salle de restaurant, bar à vin, cuisine) dont la température est supérieure à celle d'une cave de conservation du vin ;
III. la luminosité ambiante dans laquelle se trouve le dispositif (capteur de luminosité tourné vers l'extérieur de la bouteille) détecte une augmentation de luminosité supérieure à une valeur prédéterminée, par exemple 50 % : la consommation de la bouteille se fait de préférence dans un environnement plus lumineux qu'une cave de conservation du vin ;
IV. l'accéléromètre détecte une rotation de la bouteille de plus d'une valeur d'angle prédéterminée, par exemple de 270°, sur son axe longitudinal, associée à une position verticale de la bouteille dans une intervalle d'angles prédéterminés, par exemple à plus ou moins 25 degrés de la verticale, dans un mouvement dont la durée n'excède pas une durée limite prédéterminée, par exemple les six secondes, qui correspond au mouvement de la bouteille lorsque la capsule congé est découpée en vue d'ôter le bouchon de la bouteille ;
V. la bouteille est maintenue stable dans une position verticale pendant une durée prédéterminée, par exemple cinq minutes : elle est posée sur une table, par exemple, alors que la position de conservation unanimement conseillée est une position horizontale ;
VI. l'accéléromètre détecte sur une durée inférieure à une durée limite prédéterminée, par exemple une heure, une succession d'au moins un nombre prédéterminé, par exemple quatre, de positions horizontales (dans un intervalle de valeurs, par exemple plus ou moins 25°, autour de l'horizontale) non persistantes (par exemple de moins de cinq secondes) séparées par des positions verticales (dans un intervalle d'angles prédéterminé) correspondant aux moments où l'on verse le contenu de la bouteille dans des verres ;
VII. l'accéléromètre détecte un profil d'accélération du contenant similaire à celui produit par l'extraction du bouchon de la bouteille à l'aide de tout type de tire-bouchon, par exemple supérieur à 0,5 g pendant une durée inférieure une valeur prédéterminée, par exemple deux dixième de secondes ;
VIII. l'accéléromètre détecte un gabarit d'accélération du contenant similaire à celui produit par le vin qui s'écoule et l'air qui entre dans bouteille, par exemple une oscillation d'accélérations positives et négatives d'une période comprise entre 0,5 secondes et deux secondes ;
IX. la différence de mesure entre le capteur de luminosité tourné vers l'intérieur de la bouteille, après redressement de la valeur d'opacité du verre, et celle du capteur de luminosité extérieur diminue d'au moins 20% jusqu'à s'annuler lorsque le niveau de liquide baisse en deçà de la position du capteur intérieur;
X. le capteur de séparation détecte une séparation du dispositif de surveillance et de la bouteille, cette séparation pouvant avoir lieu avant la dégustation ou lors du recyclage ou de la mise au rebut de la bouteille ;

Pour les liquides peu opaques, par exemple les vins blancs, pétillants et rosés, les indices de détection peuvent, de plus, comporter :
- la température mesurée par le dispositif diminue fortement (ils doivent être rafraîchis avant leur consommation et sont donc placés dans des réfrigérateurs ou des caves de consommation dont la température est plus basse que celle d'une cave de conservation du vin)
- l'accéléromètre détecte une position non couchée de la bouteille alors que le capteur d'humidité enregistre une humidité maximale suggérant que la bouteille a été plongée dans l'eau (dans le cas des vins servis en seau pour leur conserver une température de dégustation basse).

Chacun de ces indices, observé seul, peut ne pas représenter la consommation de la bouteille. En revanche, lorsque plusieurs (par exemple au moins cinq) de ces indices sont observés dans un ordre chronologique correspondant à la consommation du liquide dans un intervalle de temps de quelques heures, la probabilité que la bouteille soit en train ou sur le point d'être consommée devient beaucoup plus forte et le dispositif réalise une détection de sortie du liquide. En cas de détection de sortie du liquide du contenant, le moyen de communication communique à distance une information représentative de cette détection.

Le serveur central reçoit toutes les informations transmises par tous les dispositifs 12 et réalise le suivi de la vie des contenants 11. On note que le serveur central peut réaliser une localisation approximative des contenants 11 par le biais des informations réseau reçues lors de la connexion du moyen de transmission.

Dans d'autres modes de réalisation, on programme, depuis un site central, la date de prochaine communication du dispositif, à chaque fois que celui-ci communique avec le site central. L'avantage de ces modes de réalisation est que l'intelligence est déportée dans le site central qui collecte les données en provenance du dispositif, les analyse et calcule la date/heure de la prochaine communication à partir de l'historique des données collectées. La mise à jour des algorithmes est facilitée car un seul site central regroupe tous les algorithmes et paramètres de tous les dispositifs connectés. En revanche, ces modes de réalisation ne laissent aucune autonomie aux dispositifs. En cas d'impossibilité de joindre le site central (panne du site ou problème de couverture réseau), le dispositif ne sait comment réagir. Dans le cas de bouteilles de vin, souvent stockées dans des caves souterraines où la couverture des réseaux radio est faible, voire inexistante, il est impossible de garantir une connectivité en permanence. Ces modes de réalisation fonctionnent alors moins bien que le mode de réalisation préférentiel présenté en regard des figures.

En ce qui concerne la réalisation pratique du dispositif 12, la sélection des composants se fait en privilégiant ceux dont la consommation au repos est la plus faible. Dans la pratique, un tableur permet de calculer une consommation théorique en fonction d'un scénario typique de vie du contenu dans son contenant (scénario où le choix serait variable en fonction du type de client et d'usage lié à une catégorie géographique ou de marché ou de niveau de qualité du contenu). Ce scénario tient compte des périodes de repos ainsi que des périodes d'activité du dispositif 12, d'échantillonnage des capteurs et de transmission de données. La donnée calculée est la consommation totale cumulée. Cet outil de calcul permet de sélectionner les composants ayant la plus faible contribution à la consommation totale dans l'usage visé, et permet de tenir compte des consommations au repos et en activité dans des proportions représentatives de la réalité. L'établissement du scénario prend en compte les types d'événements attendus dans la vie du contenant 11 en fonction des exigences du client, des exigences générales liées à la catégorie géographique du contenu, à son marché et à l'activité de ce marché, ainsi qu'au niveau de qualité attendu, notamment pour les spiritueux et le vin.

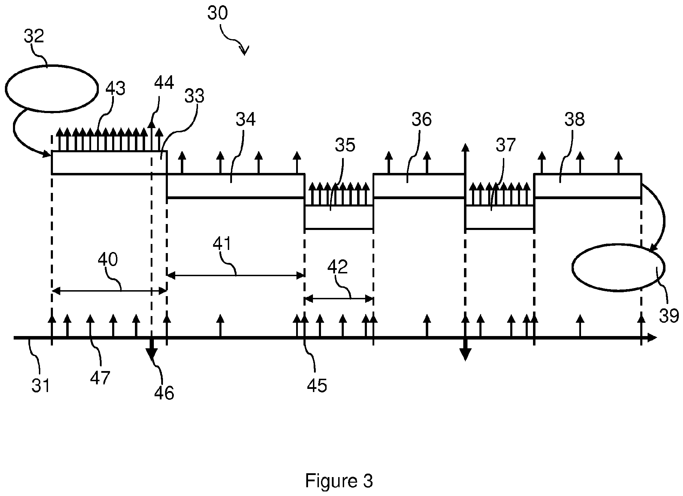

On observe, en figure 3, le fonctionnement 30 du dispositif 12 pendant une période de stockage. La ligne 31 représente le temps s'écoulant de gauche à droite. A partir de la détection du stockage 32, le dispositif 12 entre dans le mode d'apprentissage 33. Pour un contenu étant du vin, la première durée prédéterminée 40 du mode d'apprentissage 33 est, par exemple d'une à quatre semaines. Dans ce mode d'apprentissage, le dispositif 12 émet des messages 43 de capture de valeurs de paramètres d'environnement, à un premier intervalle de temps régulier. Le dispositif 12 émet aussi un signal 44 d'échantillonnage de capteur en alarme (représenté par une flèche montant de longueur supérieure à la longueur des flèches représentant les messages 43) qui provoque l'émission d'un message d'alarme 46.

Dans le mode d'apprentissage, une alarme se déclenche quand la valeur d'une grandeur mesurée sort de la plage attendue. Cette plage est soit fixée de façon arbitraire (par exemple en considérant, dans le cas du vin, des valeurs communément admises de bonne conservation du vin), soit grâce à des valeurs de cette grandeur précédemment mesurées dans les mêmes conditions, sous réserve qu'aucun changement d'état du dispositif n'ait été détecté depuis l'acquisition de ces mesures. Le message d'alarme 46 est un message transmis au serveur et dont l'objectif est d'avertir l'utilisateur final (la personne qui a la charge de la conservation du contenu) qu'une condition anormale est rencontrée et qu'une action de sa part doit être engagée pour faire cesser la condition anormale.

A partir d'un message d'état 45 (représenté par une flèche dépassant sous la ligne de temps 31) déclenché lorsque la mesure de la durée 40 atteint la première durée prédéterminée, le dispositif 12 passe en sommeil 34, pour une deuxième durée prédéterminée, par exemple de 4 à 20 semaines pour le vin. Dans ce sommeil 34, un deuxième intervalle de temps, supérieur au premier intervalle de temps, sépare deux mesures 43 de valeurs de paramètres d'environnement. A partir d'un nouveau message d'état 45, déclenché lorsque la mesure de la durée atteint la deuxième durée prédéterminée, le dispositif 12 passe en mode d'ajustement 35, similaire au mode d'apprentissage, avant de revenir à un sommeil 36, puis un nouveau mode d'ajustement 37 et un nouveau sommeil 38, jusqu'à ce que la détection d'un mouvement du dispositif 12 le fasse passer en mode de transport 39.

Les flèches 47 représentent les transmissions, au serveur central, des valeurs de paramètres d'environnement captées et mémorisées. Grâce à la mémorisation des valeurs captées, les durées des intervalles de temps entre les transmissions 47 sont supérieures aux durées des intervalles de temps entre les échantillonnages 43.

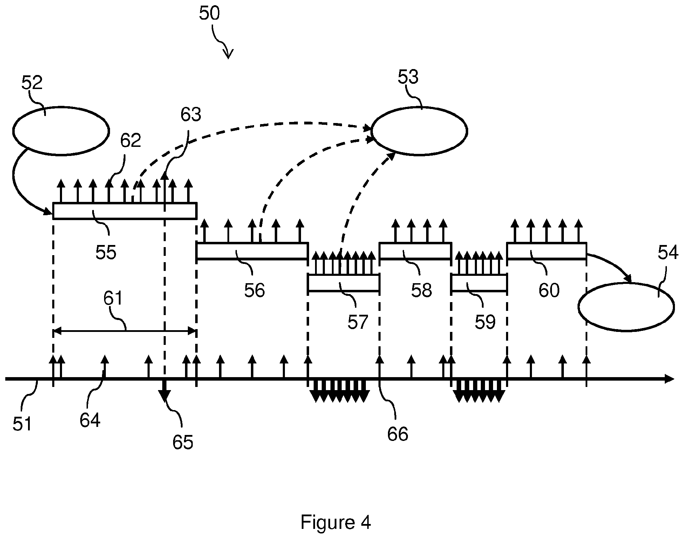

On observe, en figure 4, le fonctionnement 50 du dispositif 12 pendant une phase de transport, se trouvant en l'espèce dans un mode de transport.

Dans l'exemple du vin, le mode transport correspond à la période de la vie d'une bouteille où elle est significativement déplacée. Dans ce cas, son environnement est modifié et la bouteille rencontre des conditions environnementales qui peuvent varier rapidement et fortement. D'une façon préférentielle, dès qu'un mouvement du dispositif est détecté, le dispositif augmente sensiblement sa fréquence d'échantillonnage des valeurs des variables d'environnement et la fréquence de leur transmission au serveur central. Le milieu où se trouve le dispositif étant susceptible de varier rapidement, on ne peut se contenter de chercher à apprendre ses caractéristiques. Il faut donc les mesurer à une fréquence élevée.

L'état de transport de premier niveau (« transport1 ») est l'état dans lequel le dispositif se trouve dès que son accéléromètre est activé. Dans cet état, le dispositif étant supposé dans un environnement changeant, les fréquences d'échantillonnage et de transmission sont préférentiellement augmentées.

L'état de transport de deuxième niveau (« transport2 ») : cet état est fonctionnellement identique à l'état précédent. Seule la durée les différencie : l'état transport1 a une durée fixe alors que l'état transport de deuxième niveau dure tant qu'un mouvement est détecté par l'accéléromètre.

L'état d'alarme est l'état du dispositif lorsqu'au moins une des variables d'environnement surveillées a une valeur hors de la plage attendue. Dans ce cas, la fréquence d'échantillonnage est préférentiellement augmentée et la fréquence de transmission est préférentiellement augmentée à sa valeur maximale. Cet état dure tant qu'au moins une variable d'environnement est en dehors de la plage attendue.

Le fonctionnement pendant une phase de transport commence ainsi par une période de transport de premier niveau 55, suivant une détection 52 de mouvement du dispositif 12. La ligne 51 représente le temps, s'écoulant de gauche à droite. Pendant une troisième durée prédéterminée 61, les captures de valeurs de paramètres d'environnement 62 se succèdent à un troisième intervalle de temps prédéterminé. Le dispositif 12 émet aussi un signal 63 d'échantillonnage de capteur en alarme (représenté par une flèche montant de longueur supérieure à la longueur des flèches représentant les messages 62) qui provoque l'émission d'un message d'alarme 65. A partir d'un message d'état 66 (représenté par une flèche dépassant sous la ligne de temps 31) déclenché lorsque la mesure de la durée 61 atteint la troisième durée prédéterminée, le dispositif 12 passe dans une période de transport de deuxième niveau 56. Pendant cette période 56, un quatrième intervalle de temps, supérieur au troisième intervalle de temps, sépare deux mesures 62 de valeurs de paramètres d'environnement. A partir d'un nouveau message d'état 66, déclenché par la sortie de l'une des valeurs mesurées des intervalles de valeurs mesurés pendant la dernière période d'apprentissage ou d'ajustement, le dispositif 12 passe en mode d'alarme 57, pendant lequel préférentiellement la fréquence d'échantillonnage de chaque paramètre d'environnement et de transmission au serveur central sont à leur maximum. Lorsque toutes les valeurs captées reviennent dans les plages de valeurs calculées, un message d'état 66 fait revenir le dispositif dans une période 58 de transport de deuxième niveau, qui peut être suivie, dans les mêmes conditions d'un mode d'alarme 59 et d'une nouvelle période 60 de transport de deuxième niveau, jusqu'à ce qu'un mode de stockage soit détecté (par absence de mouvement pendant une cinquième durée prédéterminée) et ramène le dispositif en mode de stockage 54.

Les flèches 64 représentent les transmissions, au serveur central, des valeurs de paramètres d'environnement captées et mémorisées. Grâce à la mémorisation des valeurs captées, les durées des intervalles de temps entre les transmissions 64 sont supérieures aux durées des intervalles de temps entre les échantillonnages 62.

À tout moment, alors que le dispositif 12 est en mode de transport, il peut commuter en mode de consommation 53.

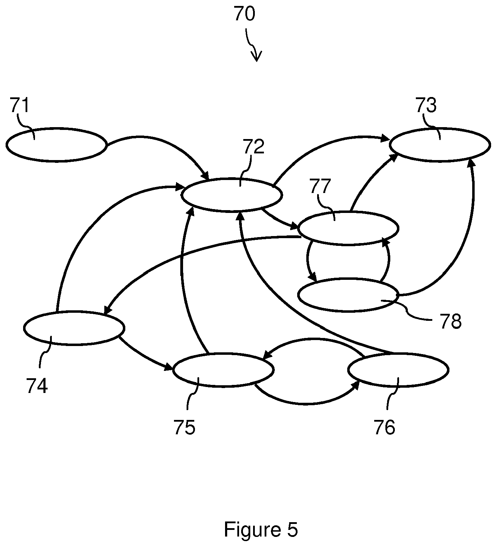

La figure 5 représente tous les états et modes que le dispositif 12 peut prendre, ainsi que les transitions entre ces états. L'état 71 est l'état d'activation. L'état 72 est le mode de transport de premier niveau. L'état 73 est l'état de consommation du contenu. L'état 74 est l'état d'apprentissage. L'état 75 est l'état de sommeil. L'état 76 est l'état d'ajustement. L'état 77 est le mode de transport de deuxième niveau. L'état 78 est l'état d'alarme. Chacun de ces états a été décrit en regard des figures 2 à 4. Les états 72, 77 et 78 se trouvent dans les périodes correspondant aux phases de transport. Les états 74, 75 et 76 se trouvent dans les périodes correspondant aux phases de stockage.

Les transitions suivantes sont commandées par des mesures de durées :
- état 74 à état 75,
- état 75 à état 76,
- état 76 à état 75 et
- état 72 à état 77.

Cependant, des valeurs des paramètres d'environnement en dehors des plages de valeurs calculées dans des états d'apprentissage 74 ou des états d'ajustement 76, peuvent provoquer les transitions suivantes :
- état 77 à état 78 ou retour de l'état 78 à l'état 77,
- état 75 à état 76.

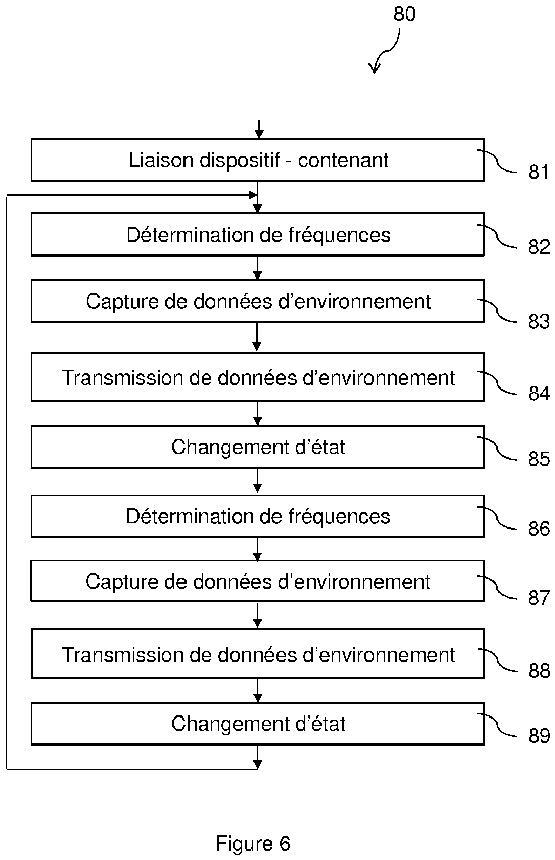

La figure 6 représente des étapes d'un mode de réalisation simple du procédé objet de l'invention. Au cours d'une étape 81, on lie le dispositif à un contenant, de manière mécanique et de manière logique (association d'identifiants dans une base de données d'un serveur distant). Au cours d'une étape 82, on détermine des premières fréquences de capture de valeurs de paramètres d'environnement et de transmission des valeurs captées. Au cours d'une étape 83, cadencée à la valeur de fréquence de capture déterminée, on capte des valeurs de paramètres d'environnement du dispositif et on mémorise ces valeurs captées. Au cours d'une étape 84, cadencée à la valeur de fréquence de transmission déterminée, on transmet au serveur distant les valeurs de paramètres d'environnement mémorisées.

Au cours d'une étape 85, on détecte un changement d'état du contenant, par exemple un mouvement ou un arrachage du dispositif. Au cours d'une étape 86, on détermine des deuxièmes fréquences de capture de valeurs de paramètres d'environnement et de transmission des valeurs captées, préférentiellement plus élevées que les premières fréquences. Au cours d'une étape 87, cadencée à la valeur de fréquence de capture déterminée, on capte des valeurs de paramètres d'environnement du dispositif et on mémorise ces valeurs captées. Au cours d'une étape 88, cadencée à la valeur de fréquence de transmission déterminée, on transmet au serveur distant les valeurs de paramètres d'environnement mémorisées. Au cours d'une étape 89, on détecte un changement d'état du contenant, par exemple un arrêt de mouvement et on retourne à l'étape 82.

Préférentiellement, le procédé comporte aussi (les étapes ci-dessous ne sont pas représentées en figure 6) :
- une étape d'apprentissage de plages de valeurs de grandeurs physiques captées pendant une période prédéterminée suivant un détection de changement d'état, période ne comportant pas de détection de changement d'état et une étape de transmission d'un signal d'alarme, après cette période d'apprentissage, lorsqu'au moins une valeur de grandeur physique captée se trouve en dehors de la plage de grandeurs physiques apprise ;
   et/ou
- une étape de détection de la sortie du liquide du contenant (11) et une étape de communication d'une information représentative de la détection de la sortie du liquide.

L'objet des algorithmes est de détecter les phases de la vie du contenant et le passage d'une phase à l'autre, afin de déterminer le comportement du dispositif qui varie durant chacune de ces phases ou à l'intérieur même de chaque phase : dans chaque phase, plusieurs états peuvent se succéder avec, pour chaque état, un comportement particulier et prévisible du dispositif qui concerne la façon dont il utilise ses capteurs et périphériques pour mesurer ses conditions d'environnement ou de transport et son système de transmission pour transférer ces informations à un système central de traitement et d'alerte.

D'une manière générale, le mode de consommation et de fin de vie du contenant correspond à la phase terminale de la vie du dispositif. Le traitement des valeurs remontées par les capteurs permet de détecter avec une bonne probabilité la consommation du contenu. La consommation s'accompagne également de la séparation du dispositif et du contenant (pour rendre le recyclage possible). Dans ce dernier cas, le capteur de séparation permet de détecter la fin de vie du dispositif.

La présente invention fournit :
- une capacité à spécialiser les dispositifs en fonction de certains paramètres spécifiques de besoins métier, en adaptant au sein d'un même groupe de dispositifs le software ou le hardware embarqué des contenants faisant partie de ce groupe,
- une capacité à choisir la capacité de la pile en fonction de l'autonomie désirée pour le système,
- une capacité à rendre mobiles des applications qui étaient fixes par nécessité de disposer d'une source de courant à proximité,
- une capacité à superviser à distance les conditions d'environnement et de mouvement d'objets potentiellement mobiles,
- une capacté à détecter certains états du contenant par l'interprétation des données des capteurs (exemple : consommation du contenu via la température, le mouvement, la position verticale, la luminosité interne et externe),
- une localisation approximative des contenants par le biais des informations réseau lors de la connexion du système de transmission,
- une capacité à construire une historique des positions approximatives, permettant ainsi de retracer la vie du dispositif (un contenant) ou d'une collection de dispositifs (caisses, palettes, etc.) par ses mouvements, ses étapes/ modes de transport, par pays, par régions, ... et
- une capacité à retracer statistiquement les données marketing et commerciales issues des contenants : estimation du nombre de contenants d'une série donnée de fabrication non consommés, par exemple dans le cas du vin d'un millésime / château / cuvée encore en circulation, rareté relative par marché / pays / région, habitudes de consommation par marché / pays / région.

Dans le domaine du vin, la présente invention rend possibles les applications suivantes en modifiant marginalement le serveur ou l'interface homme/machine :
- Système antivol pour le vin : détection du mouvement de bouteilles de vin équipées du dispositif, par exemple lors d'un vol ;
- Surveillance de caves et d'espaces de stockage de vin : détection des conditions de stockage dans une cave et remontée d'alarmes quant aux variables d'environnement, en partant du principe que les paramètres d'environnement et de mouvement déterminent les conditions d'environnement de la bouteille équipée du dispositif, mais également de toute autre bouteille dans le voisinage immédiat de la bouteille équipée.
- Surveillance des systèmes dits « cave à vin » (de vieillissement ou de service) qui créent par un système de climatisation à compresseur et d'humidification les conditions supposées idéales pour la conservation ou la dégustation du vin. Le dispositif peut, dans ce cas, alerter l'utilisateur de toute panne de la cave à vin (remontée des températures, humidité non appropriée, ...) mais également, en dehors de toute panne de la cave à vin, du niveau des vibrations engendrées par le moteur du compresseur et encaissées par la bouteille équipée du dispositif ;
- Gestion automatique de cave : chaque bouteille équipée du dispositif permet de tenir automatiquement à jour un registre de cave : date d'acquisition des bouteilles, durée et conditions de conservation ainsi que date de consommation sont renseignées automatiquement par les données remontées par le dispositif sur le serveur central.

Dans des modes de réalisation, on rend le dispositif moins gourmand en énergie avec une portée beaucoup plus réduite en y ajoutant des répéteurs/ passerelles dans les caves, les zones non couvertes par des réseaux radio et dans tous les lieux de transport/ stockage (par exemple, selon le protocole Bluetooth, marque déposée), en ajoutant de la mémoire permettant en l'absence de connexion de stocker les données au fur et à mesure jusqu'à la prochaine connexion effective.

Comme on le comprend à la lecture de la description qui précède, la mise en oeuvre de la présente invention permet de garantir la sécurité, l'authenticité et l'intégrité du processus et des données : celle de l'association objet/contenant, du traitement et des données transmises depuis la mesure (capteur analogique) jusqu'au terminal déporté du monitoring. Dans ce cadre, le dispositif surveille des valeurs de paramètres d'environnement et de mouvement de façon pertinente.

L'authenticité, l'intégrité et la protection contre toute intrusion volontaire des données relatives à la surveillance de l'environnement et des mouvements sont garanties par la présence d'un capteur complémentaire, ainsi que d'un deuxième capteur complémentaire lorsque le contenant est translucide ou, au moins partiellement, transparent : un contacteur qui détecte toute tentative de séparation frauduleuse du dispositif et du contenant et, lorsque le contenant est translucide ou, au moins partiellement, transparent, un capteur de luminosité dirigé vers l'intérieur du contenant qui détecte un changement d'environnement suspect en cas de déplacement frauduleux du contenant.

Dans le cas du vin, l'encombrement du dispositif 12 est faible : le dispositif 12 est un ajout unitaire pour chaque bouteille, compatible avec les conditionnements usuels (cartons, caisses, ...) et ne nécessitant aucune modification de ces conditionnements ni de la bouteille. D'une manière pratique, l'encombrement et le poids faibles se traduisent par exemple par la conformation du dispositif en bracelet placé autour de la bouteille et d'une épaisseur variant sur une bouteille entre trois et 25 mm selon les configurations ou encore exploitant certains creux ou aspérités tels que la piqûre de la bouteille.

## Revendications

1. Dispositif (12) de surveillance d'un liquide contenu dans un contenant (11), **caractérisé en ce qu'**il comporte :
- un capteur (13) d'au moins une valeur de grandeur physique d'environnement du contenu, fournissant, à une fréquence d'échantillonnage, des valeurs captées,
- un moyen (14) de communication, à une fréquence de transmission, d'information représentative des valeurs captées de chaque grandeur physique,
- un capteur (15) d'état du contenant configuré pour détecter un changement d'état du contenant, et
- un contrôleur (16) configuré :
- pour réaliser un apprentissage de plages de valeurs de grandeurs physiques captées pendant une période prédéterminée suivant un détection de changement d'état, période ne comportant pas de détection de changement d'état, le moyen de communication étant configuré pour transmettre un signal d'alarme, après cette période d'apprentissage, lorsqu'au moins une valeur de grandeur physique captée se trouve en dehors de la plage de grandeurs physiques apprise ;
et / où
- pour détecter la sortie du liquide du contenant (11), le moyen de communication étant configuré pour transmettre une information représentative de la détection de la sortie du liquide.

2. Dispositif (12) selon la revendication 1, dans lequel le contrôleur (16) est configuré pour changer de fréquence d'échantillonnage et/ou de fréquence de transmission après détection d'un changement d'état par le capteur d'état.

3. Dispositif (12) selon l'une des revendications 1 ou 2, qui comporte une horloge (18) pour fournir au moyen de communication (14) la fréquence de transmission et une alimentation électrique (17) configurée pour n'alimenter de manière continue que le contrôleur (16), le capteur (15) d'état et l'horloge, chaque capteur (13) de valeurs de grandeur physique et le moyen de communication n'étant alimentés que par intermittence.

4. Dispositif (12) selon la revendication 3, dans lequel l'alimentation électrique (17) est une pile au lithium - chlorure de thionyle et/ou présente les caractéristiques suivantes :
- elle présente une consommation permanente inférieure à 10 nA ;
- elle supporte des pics de courant de 50mA et
- elle ne présente pas d'autodécharge.

5. Dispositif (12) selon l'une des revendications 1 à 4, dans lequel le capteur (15) d'état est configuré pour détecter un déplacement du contenant.

6. Dispositif (12) selon l'une des revendications 1 à 5, dans lequel le capteur (15) d'état comporte un accéléromètre.

7. Dispositif (12) selon l'une des revendications 1 à 6, dans lequel le capteur (15) d'état est configuré pour détecter la séparation du dispositif, d'une part, du contenant (11), d'autre part.

8. Dispositif (12) selon l'une des revendications 1 à 7, dans lequel le contrôleur (16) est configuré pour augmenter la fréquence d'échantillonnage et la fréquence de transmission après détection d'un déplacement du contenant.

9. Dispositif (12) selon l'une des revendications 1 à 8, dans lequel le contrôleur (16) est configuré :
- pour réaliser un apprentissage de plages de valeurs de grandeurs physiques captées pendant une période prédéterminée suivant un détection de changement d'état, période ne comportant pas de détection de changement d'état et
- pour transmettre un signal d'alarme, après cette période d'apprentissage, lorsqu'au moins une valeur de grandeur physique captée se trouve en dehors de la plage de grandeurs physiques apprise.

10. Dispositif (12) selon la revendication 9, dans lequel l'apprentissage est réalisé en traitant statistiquement les valeurs captées pendant la période prédéterminée suivant un détection de changement d'état, période ne comportant pas de détection de changement d'état, la plage de valeurs s'étendant, pour au moins une grandeur physique de la valeur moyenne moins un premier nombre prédéterminé de fois l'écart-type jusqu'à la valeur moyenne plus un deuxième nombre prédéterminé de fois l'écart-type.

11. Dispositif (12) selon l'une des revendications 9 ou 10, dans lequel le contrôleur est configuré pour déterminer, pour une combinaison de valeurs captées de grandeurs physiques sensiblement constante pendant l'année, une plage de valeurs ne déclenchant pas la transmission d'un signal d'alarme.

12. Dispositif (12) selon l'une des revendications 1 à 11, dans lequel le capteur (13) d'au moins une valeur de grandeur physique d'environnement du contenu (11) comporte des capteurs de valeurs de grandeurs physiques représentative de la température, de l'hygrométrie, de l'éclairement et de la pression atmosphérique de l'environnement du contenant.

13. Dispositif (12) selon l'une des revendications 1 à 12, dans lequel le contrôleur (16) est configuré pour détecter la sortie du liquide du contenant (11), le moyen (14) de communication étant configuré pour transmettre une information représentative de la détection de la sortie du liquide.

14. Procédé (80) de surveillance d'un liquide contenu dans un contenant, **caractérisé en ce qu'**il comporte :
- une étape (83, 87) de capture d'au moins une valeur de grandeur physique d'environnement du contenu, fournissant, à une fréquence d'échantillonnage, des valeurs captées,
- une étape (84, 88) de communication, à une fréquence de transmission, d'information représentative des valeurs captées de chaque grandeur physique,
- une étape (85, 89) de capture d'état du contenant configuré pour détecter un changement d'état du contenant ; et
- une étape d'apprentissage de plages de valeurs de grandeurs physiques captées pendant une période prédéterminée suivant un détection de changement d'état, période ne comportant pas de détection de changement d'état et une étape de transmission d'un signal d'alarme, après cette période d'apprentissage, lorsqu'au moins une valeur de grandeur physique captée se trouve en dehors de la plage de grandeurs physiques apprise ;
et/ou
- une étape de détection de la sortie du liquide du contenant (11) et une étape de communication d'une information représentative de la détection de la sortie du liquide.

15. Procédé selon la revendication 14, qui comporte, de plus, une étape (82, 86) de changement de fréquence d'échantillonnage et/ou de fréquence de transmission après détection d'un changement d'état.
